# EUROPEAN PATENT SPECIFICATION

(11) **EP 2 627 400 B1**
(45) Date of publication and mention of the grant of the patent: **06.04.2016**
(21) Application number: 11833960.5
(22) Date of filing: 06.10.2011
(51) Int. Cl.: A61N 1/04

(54) **A DISPOSABLE PATCH FOR PERSONAL AESTHETIC SKIN TREATMENT**
EINWEGPFLASTER ZUR ÄSTHETISCHEN HAUTBEHANDLUNG
TIMBRE À USAGE UNIQUE POUR TRAITEMENT CUTANÉ ESTHÉTIQUE PERSONNEL

(30) Priority: 27.12.2010 US 201061427305 P; 17.10.2010 US 393902 P
(43) Date of publication of application: 21.08.2013
(73) Proprietor: Syneron Medical Ltd., 20692 Yoqneam Illit (IL)
(72) Inventor: ECKHOUSE, Shimon, 34980 Haifa (IL); FLYASH, Lion, 17512 Nazaret Illit (IL)
(74) Representative: O'Neill, Brian
(86) International application number: PCT/IL2011/000781
(87) International publication number: WO 2012/052986

(56) References cited:
- WO-A1-2012/073232
- WO-A2-2012/052986
- US-A1- 2006 009 691
- US-A1- 2006 190 057
- US-A1- 2006 276 858
- US-A1- 2009 048 651
- US-A1- 2009 270 788
- US-A1- 2009 299 361
- US-A1- 2009 299 361
- US-A1- 2010 004 715

## Description

### TECHNOLOGY FIELD

The present apparatus is related to the field of personal aesthetic procedures and in particular to cosmetic skin treatment procedures.

### BACKGROUND

Skin tightening or wrinkle reduction, removal of skin lesions and blemishes, reduction of subcutaneous fat or adipose tissue, are aesthetic treatments for which there is a growing demand. Types of available aesthetic therapy commonly include the application of different light sources, radio frequency energy and sometimes ultrasound energy.

The electromagnetic energy is typically delivered to a target segment of the skin of a recipient by selecting a contact element that is compatible with the treated skin segment size. Alternatively, a plurality of contact elements may be utilized, in which the plurality of elements contact discrete points of the target segment of the skin. In the latter case, the healing period is typically shorter. Although both modes of treatment are effective, the use of multiple contact elements treating discrete points or fractions of a target skin segment effectively tightens the skin, reduces wrinkles, and improves the skin appearance. In recent years, non-invasive, non-ablative aesthetic skin treatments have been introduced and may replace ablative skin treatment procedures in the future. In non-ablative skin treatment, thermal energy induces certain tissue modification and in particular collagen modification in the dermis. Currently non-ablative skin treatment is used for skin tightening, scar removal, acne treatment, and other aesthetic procedures typically performed in an ambulatory environment.

In non-ablative skin treatment radiofrequency (RF) energy, depending on the spacing of electrodes, is deposited 100-2500 µm below the skin surface, where the energy does not affect the epidermis and the skin layer in which most of the skin aging processes occur. With no epidermal wound, there is almost no recovery period and thus no interruption of daily life routines. Transient erythema or mild edema, are the only known side effects and those disappear a few hours after the treatment. The efficiency of the non-ablative treatments is lower than the one of ablative treatments; however, non-ablative skin treatments also stimulate new collagen production and repair tissue defects.

Since there are no side effects and the procedure does not leave wounds requiring a long healing period, the non-ablative treatment is associated with little or no downtime and unlike the ablative skin treatment, which requires professional supervision, non-ablative skin treatment may be used by a lay user in a home environment at a time most convenient for him/her to perform a treatment session such as, for example, skin tightening and wrinkle reduction associated with collagen remodeling.

RF energy is conducted to skin through electrodes. With proper design of RF applying electrodes, RF energy power setting and application time the energy may be accurately conducted to the desired target tissue. For example, the energy application time and power may be shorter than skin thermal relaxation time further simplifying the non-ablative skin treatment. The employment of an applicator that includes disposable parts for electromagnetic radiation skin treatment also simplifies and facilitates aesthetic treatments in a home environment at a time most convenient for the user to perform a treatment session.

Use of RF energy for performing skin treatment on a lay user in a residential environment as compared to professional use devices requires increased safety, reduced device size and freedom to perform concurrently to the treatment other tasks.

US2006/190057 discloses a device for transcutaneous electrical nerve stimulation (TENS) adapted to create a traveling wave of electrical pulses. The TENS therapy may be combined with additional stimulation therapies such as heat or vibration.

### BRIEF SUMMARY

The invention is defined by independent claim 1. Preferred embodiments are defined by the dependent claims. The treatment RF current generated by the applied RF voltage heats the skin and is applied intermittently to different electrodes being in contact with the skin in an order and duration sufficient to cause the desired skin effect and enable proper cooling of earlier treated skin segments. The selected protocol ensures safe non-ablative skin treatment parameters.

Typically, the electrodes are assembled on a common substrate or carrier that may be a reusable or disposable carrier. One or more light sources providing illumination to the treated skin segment may be assembled on the same substrate. The light sources may be operative to illuminate the treated skin segment independent of the RF voltage application, concurrent with the RF voltage application or in sequentially with RF voltage application.

### GLOSSARY

The term "patch" in the context of the present disclosure means a substrate having an array of voltage to skin application elements or electrodes. The electrodes may be in the form of one or more rows of voltage to skin application elements, a two dimensional array or matrix of voltage to skin application elements and a three-dimensional shape substrate having on the surface to be applied to the skin voltage to skin application elements. In addition to the electrodes the patch may include light source, for example surface mounted LEDs or fiber optics lines.

The terms "electrodes", "conductive elements", "contact elements" and "voltage to skin application elements" are used interchangeably in the present disclosure and mean elements operative to receive voltage from a source such as, for example, an RF voltage generator and apply the received voltage to the skin.

The term "skin treatment" as used in the present disclosure includes cosmetic treatment of various skin layers such as stratum corneum, dermis, epidermis, skin rejuvenation procedures, pigmented lesions removal, acne treatment, and such procedures as collagen shrinking or destruction. The terms "RF voltage" and "RF power" are used interchangeably in the present disclosure. The mathematical relation between these two parameters is well known and knowledge of the value of one of them enables easy determination of the value of the other parameter.

### BRIEF DESCRIPTION OF THE DRAWINGS

Various embodiments of the present apparatus are disclosed and presented, by way of non-limiting examples only, with reference to the accompanying drawings, wherein like numerals depict the same elements throughout the text of the specifications. The present apparatus will be understood and appreciated more fully from the following detailed description, taken in conjunction with the drawings in which:
FIG. 1 is a simplified illustration of an exemplary embodiment of the present apparatus for personal aesthetic skin treatment;
FIGS. 2A, 2B, 2C and 2D are plan view simplified illustrations of some exemplary embodiments of disposable RF to skin application patches;
FIG. 3 is a planar view simplified illustration of a sheet of electrodes containing a number of exemplary patch shapes in accordance with the present apparatus;
FIGS. 4A and 4B are simplified illustrations of an exemplary embodiment of cosmetic skin treatment in accordance with the present apparatus;
FIGS. 5A and 5B are frontal and rear planar views illustrating an additional exemplary embodiment of a disposable skin treatment patch in accordance with the present apparatus;
FIGS. 6A and 6B are plan view simplified illustrations of another exemplary embodiment of disposable RF to skin application patch;
FIGS. 7A, 7B, 7C and 7D are plan view simplified illustrations of generation of a linear sweeping heating wave effect according to an exemplary embodiment of the present apparatus; and
FIG. 8 is a simplified illustration of a user performing skin treatment in accordance with the current apparatus.

### DETAILED DESCRIPTION OF EXEMPLARY EMBODIMENTS

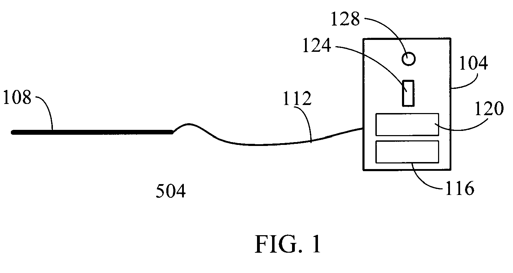

Reference is made to FIG. 1, which is a simplified illustration of an exemplary embodiment of the present apparatus for personal aesthetic skin treatment. As shown in FIG. 1, apparatus 100 includes a case 104, a disposable RF to skin application patch 108 and a cable 112 connecting between case 104 and patch 108. Case 104 may contain a source of power 116, an RF voltage generator 120, an ON-OFF switch or button 124, and an operation status indicator 128 such as a Light Emitting Diode (LED) that may lit with one or more colors. The ON-OFF switch may include more than one button enabling selection of a number of treatment protocols. The source of power 116 may be one or more of conventional batteries that are disposed upon depletion or one or more of rechargeable batteries. The distal end of cable 112 is equipped by a fast release connector (not shown) enabling easy connection between case 104 and disposable RF to skin application patch 108.

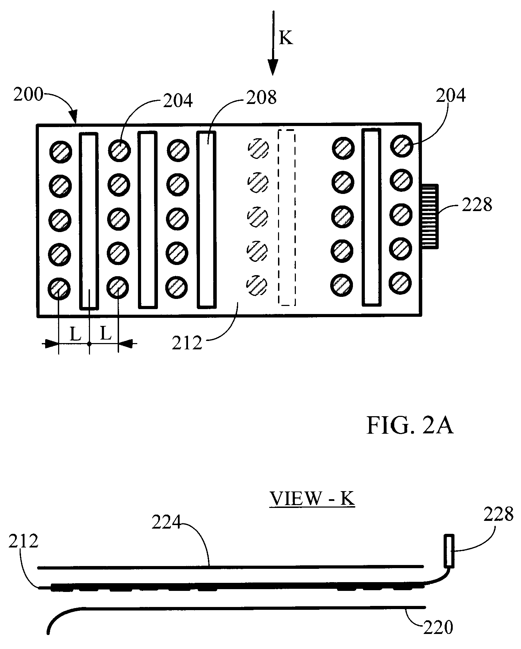

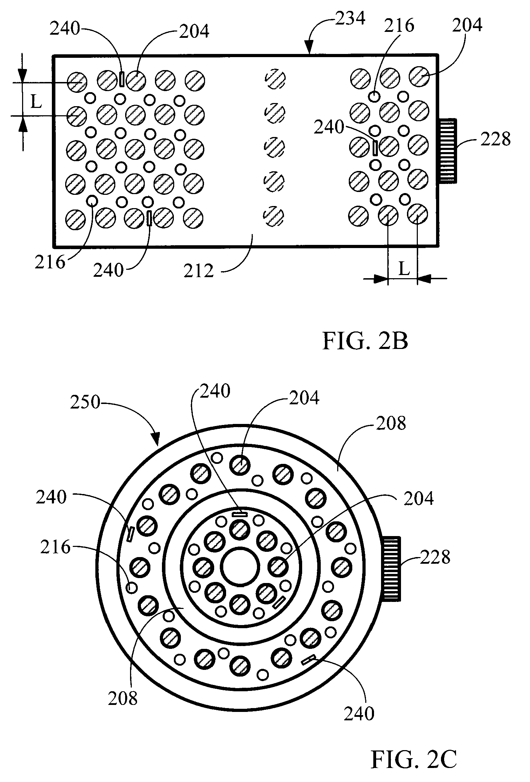

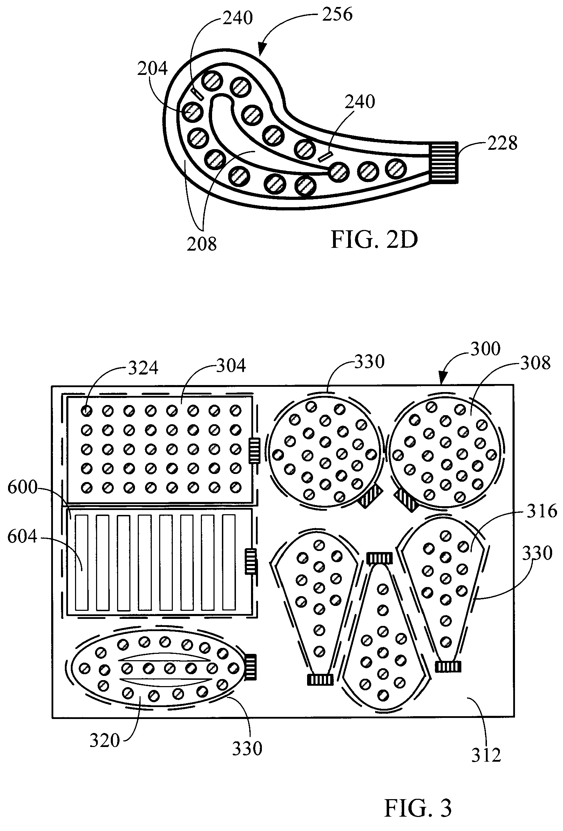

FIGS. 2A, 2B, 2C and 2D are plan view simplified illustrations of some exemplary embodiments of disposable RF to skin application patches. FIG. 2A illustrates a rectangular patch 200 that may be used instead of patch 108. Generally, patches 108, 200 and other patches to be described below are multilayer structures where one or more electrodes or voltage to skin application elements 204 and 208 are deposited on a substrate 212. The voltage to skin application elements or electrodes 204 and 208 may be coated by a coating enhancing certain electrode surface properties. For example, the coating may be an adhesive coating including an electrically conductive biocompatible adhesive, for example, such as Conductive Adhesives for Biomedical Electrodes commercially available from First Water Limited, Ramsbury, Wiltshire SN8 2RB U.K. or conductive, pressure sensitive adhesive commercially available from Avery Dennison, Inc., Painesville, Ohio 44077 U.S.A. Such adhesive would enable firm electrical and mechanical coupling of the electrode to the skin when the patch is applied to the skin. A release layer 220, which may be a suitable paper or plastic material, protecting the adhesive may be attached to the electrodes. The release layer may cover the whole surface of the patch. The other side, of substrate 212 on which electrodes interconnecting pattern is deposited, may be covered by a protective layer 224. Protective layer 224 may be a plastic or paper layer of a desired color or a lacquer layer. The color of the protective layer may be used as a code indicating patch 200 skin treatment parameters.

In another embodiment protective layer 224 may be a thermally conductive material, since some of the heat developed at the skin-electrode contact surface will be conducted through the electrodes to the conductors interconnecting pattern deposited on the opposite to the electrodes side of the substrate. Such material, with proper electrical isolation means, may be aluminum or copper foil, metal powder included in the material of layer 224 or similar materials. The heat dissipation capability of the foil may be enhanced by providing a structure of fine ribs or fine rough structure of the heat dissipating surface of coating layer 224. At least one edge of substrate 212 may have an extension 228 or a bay enabling easy and quick connection to RF voltage generator 124 located in case 104 (FIG. 1) by cable 112.

Patch 200 of FIG. 2A has electrodes layout where one or more electrodes 204 located on both sides of a common electrode 208. This electrodes layout enables patch operation is similar to mono-polar RF operation mode. The spacing between electrodes 204 and 208 is uniform and electrodes 204 located on both sides of electrode 208 may be located on the same distance from electrode 208. Optionally, the spacing between the electrodes may be coated by an electrically insulating adhesive, which ensures better patch to skin adhesion and stability. Uniform spacing ***L*** between the electrodes enables treatment of all skin segments, actually skin volumes located below the skin segments surface, to which the patch is applied at the same skin treatment depth. For treatment of skin layers located at other skin depth patches with different spacing or distance between the electrodes may be used. Alternatively, a different electrode switching pattern enabling treatment of skin layers located at different depth may be implemented.

FIG. 2B illustrates a patch 234 configured to operate in bi-polar operation mode. Common electrodes 208 have been replaced by electrodes 204 located on a grid having equal step/spacing *L* in both grid directions, although other asymmetric electrodes spacing is possible. Optionally, surface mounted LEDs 216 may be incorporated in the patch. Alternatively, a LED or other suitable light source may be included into case 104 and a fiber optic bundle, which may be incorporated into cable 112, may guide the light to the treated skin segment. LEDs 216 or the light source may emit radiation with wavelength of 570 -780nm.

Voltage to skin applying elements or electrodes may be produced by different methods. Typically, methods used in printed circuit board production may be suitable for voltage to skin applying elements or electrodes production. These methods enable low cost production of a large amount of substrates populated by electrodes. Depending on the type of processing and material deposition the voltage to skin applying elements may be flat, protruding from the surface on few microns or more as desired. By proper selection of the metal deposition process the voltage to skin applying elements may be made flat or have a certain shape and surface texture. The substrate 212 on which the electrodes 204 and 208 and LEDs 216 reside is common to all electrodes and may be made of a variety of materials, typically insulating materials. Non-limiting example of a suitable substrate materials include polyimide film, paper, or similar material, with a thickness of 0.5mil to 60mil (12.5 micron to 1500 micron). All described above patches are configured to include an extension 228 or similar connector type arrangement allowing quick attachment to apparatus 100.

The patches may be of different geometrical shapes and sizes. The shape of the patches may resemble the skin segment to which the patches may be applied, e.g. under eyes, on the neck, etc. FIG. 2C illustrates a round shape patch 250. The patch may be configured to operate in bi-polar operation mode also. Common electrodes 208 could be replaced by electrodes 204 located on a grid having equal or different step in both grid directions. FIG. 2D illustrates a patch 256 having a shape suitable for example, for application under eyes.

In some embodiments, each of the patches may include one or more temperature sensors 240, which may be a thermistor, a thermocouple or a thin film sensor.

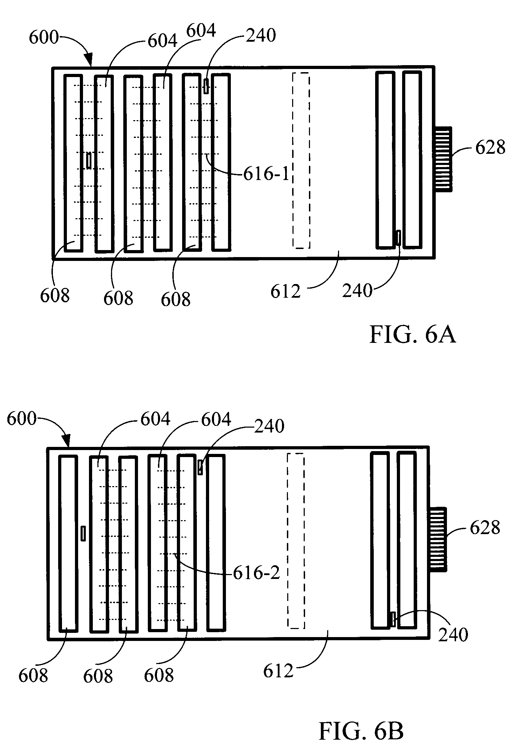

The patches for personal fractal cosmetic skin treatment may be supplied to the user in single units according to the desired predetermined patch shape. Alternatively, as shown in FIG. 3, the patches may be supplied in substrate sheets 300. The substrates 312 of sheets 300 are made of the same material as substrate 212 and each substrate may contain a plurality of different or identical patch shapes and sizes, for example, such shapes as shapes 304, 308, 316, and 320. Different or identical electrode 324 configurations may be placed on each of the patch shapes. (For the simplicity of the explanation all of the patches, except patch 600, are shown with similar electrodes.) A layer of electrically conductive adhesive (not shown) covers the surface of each of the located on the patch electrodes. The space between electrodes may be covered by an electrically isolating adhesive. A release layer may cover the sheet of disposable patches. Cut-out lines 330 enabling easy patch 304, 308, 316, 320 or 600 (FIG. 6) from sheet 300 separation could be made in the substrate 312 and in the release layer. Supply of skin treatment patches in sheets containing a plurality of shapes reduces distribution and manufacturing cost and provides the user with a greater patch selection freedom.

The size of the patches may vary and may be adapted to the size of treated skin segment. The patches may be small for example 10mmX10mm for localized skin treatment or large enough, for example 100mm by 100mm to treat relatively large skin segments.

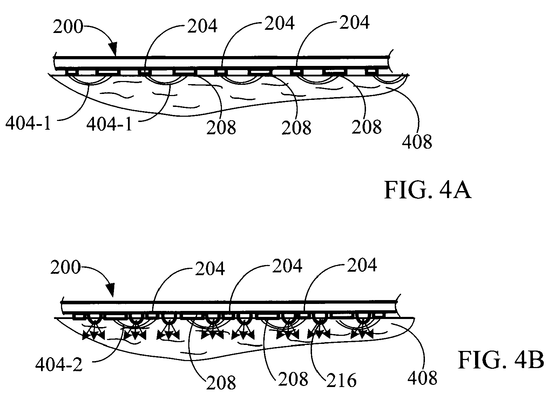

FIGS. 4A and 4B are simplified illustrations of an exemplary embodiment of cosmetic skin treatment in accordance with the present apparatus. Initially the user applies patch 200, or any other of the patches described above, to a segment of the user skin to be treated and checks that the electrodes are in firm contact with the skin. The user connects between patch 200 and case 104 by cable 112 and by pushing button 124 (FIG. 1) switches ON RF voltage generator 120 and may set one of the predetermined skin treatment protocols. RF voltage generator provides test RF voltage and determines the quality of the contact between the patch electrodes and the treated segment of skin. Upon determination of the electrodes status RF voltage generator may begin delivery of the treatment RF voltage to the electrodes of the patch. The user may select a proper treatment protocol by using the ON-OFF switch and keying in the protocol number.

The magnitude of test RF voltage supplied to the electrodes is set between 10vrms to 30vrms and of the treatment voltage between 20vrms to 200vrms such as not to cause any excessive and damaging skin heating. The thermal properties of the skin are sufficiently predictable; the effect of treatment can be estimated from previous measurements made in laboratory conditions. These measurements may be a basis for predetermined skin treatment protocols according to which the RF voltage generator will operate. The lengths of the intervals (and the time between the intervals) in course of which RF voltage generator 120 (FIG. 1) may deliver RF voltage and induce in skin 408 RF current could be controlled without having direct temperature feedback by "dosing" the intervals or pulse length of the RF voltage. For example, the pulses may have duration of 0.5sec to 4sec or even be set to operate in pseudo continuous mode. Proper dosing may be included in each of the predetermined skin treatment protocols. Alternatively, temperature sensors 240 (FIG. 2) may be operative to switch OFF the supply of RF voltage when the skin or electrode temperature exceeds the desired or preset limit.

In order to further mitigate potential skin overheating, initially RF voltage may be delivered to common electrodes 208 (FIGS. 2A, 2B, 2C and 2D) and to all fractal electrodes located on one side (for example, on the left or first side or in the inner circle (FIG. 2C) of the common electrode 208 creating in the skin 408 an electric current schematically shown by lines 404-1 (FIG. 4A).

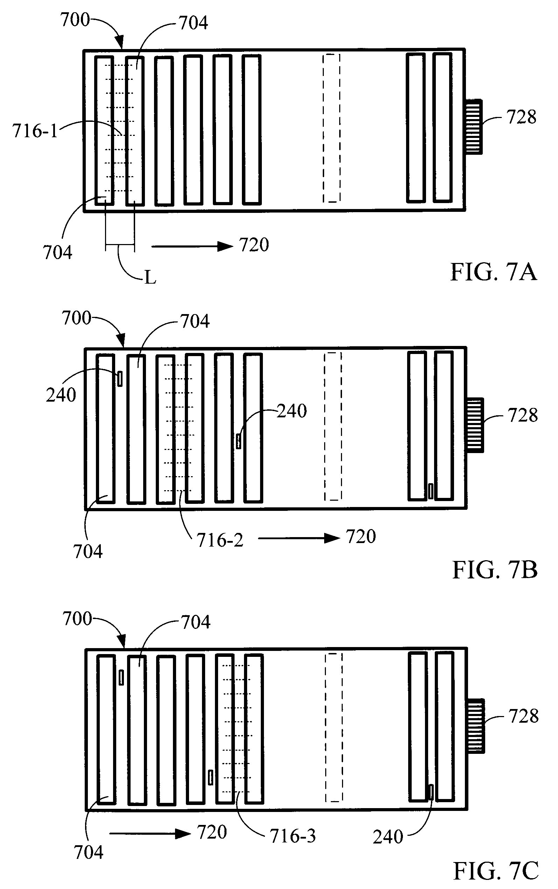

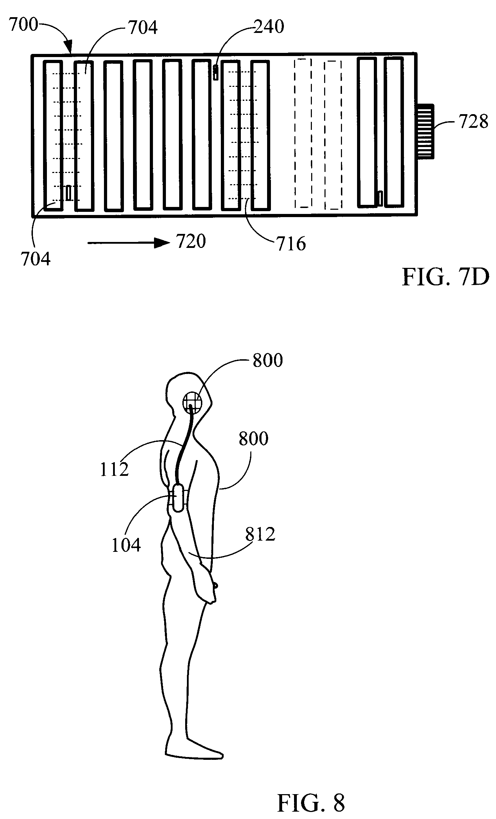

Upon completion of delivering of RF voltage to the first group of electrodes, RF voltage generator may switch-off the first group of electrodes and begin delivery of the RF voltage to the same common electrodes 208 and another group of fractal electrodes 204, for example, electrodes located on the right or second side or in the outer circle (FIG. 2C) of the common electrode 208 creating in the skin 408 an electric current schematically shown by lines 404-2 (FIG. 4B). This mode of treatment enables fractal treatment of densely placed skin treatment points and reduces the risk of skin overheating. The earlier treated fractions of skin are thermally relaxing or cooling when the next fraction of skin is treated. (Skin thermal relaxation time is known to vary from few milliseconds to few seconds depending on the depth of the treatment.) FIG. 4B illustrates optional LEDs 216 mounted on patch 200 and operative to illuminate the treated segment of skin 408. Alternatively, terminations of fiber optics guides emitting light from a source of illumination may be mounted on patch 200. LEDs 216 or fiber optics terminations may illuminate the treated segment of skin 408 concurrently with the application of RF voltage, before the application of the RF voltage or after the application of RF voltage. Coating layer 224 assists in reducing temperature of the skin surface. A similar mode or operation may be applied to patches with fractal only electrodes (FIG. 2B) or conventional electrodes 604 and 704 (FIG. 6 and FIG. 7).

Patch electrodes 204 and 208 (FIG. 2) are in permanent engagement or contact with skin while the RF current is supplied in pulses to increase the temperature of the treated skin segments or volumes under the skin surface to about 60 - 62 degrees Celsius and maintain it for a certain treatment time, although limiting heating of the skin surface to 40 - 45 degrees Celsius or a lower value. As indicated earlier the treatment mode and treatment parameters may be determined earlier in laboratory conditions and applied/configured by the patch user. Although, the treatment parameters may be determined earlier in laboratory conditions and applied by the patch user, temperature sensors 240 may be activated to control the skin and electrode temperature and if necessary switch-OFF RF voltage to electrodes supply.

In use case 104 may be placed in a pouch located on the user waist or hand, similar to the way iPod and other music playing apparatuses are carried. This enables complete user freedom that in course of treatment may address and work on other issues and tasks.

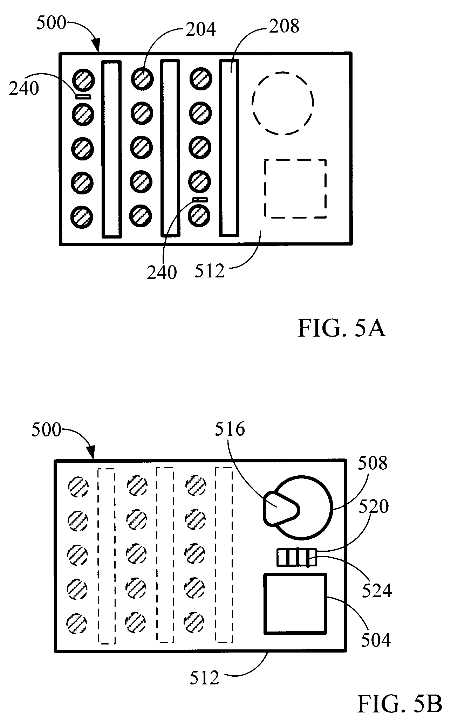

FIG. 5A and 5B are frontal and rear planar views illustrating an additional exemplary embodiment of a disposable skin treatment patch in accordance with the present apparatus. Depending on the amount of electrodes, patch 500 that may be used instead of patch 104 or 200 or any other described above patches. Patch 500 and any other described in the present disclosure patch may be implemented as a disposable patch. The patch could include an RF voltage generator 504 and a power source such as a battery 508 and as such the patch becomes an autonomous patch that does not require connection to a power supply. The electrodes 204 and 208 of the patch 500 are coated by an electrically conductive adhesive enabling a firm electrical and mechanical coupling of the electrode to the skin. When patch 500 is applied to skin a current begins flowing in the skin. The current is sensed by a current sensor and if all of the electrodes are in firm contact with the skin, it switches ON RF voltage generator 504 and supplies treatment voltage to the skin.

RF voltage generator 504 of patch 500 may operate according to a single or a number of skin treatment protocols. When patch 500 is designed to operate in a number of skin treatment protocols it includes an optional skin treatment protocol setting device 520. In order to instruct the patch/RF voltage generator to operate the desired skin treatment protocol, the user configures the skin treatment protocol setting device 520 by simply cutting one or more conductors 524 leaving the one or a combination of conductors that enables the desired skin treatment protocol. As a safety measure temperature sensors 240 may be mounted on patch 500 substrate and used to switch-OFF RF voltage supply.

FIGS. 6A and 6B are plan view simplified illustrations of another exemplary embodiment of a disposable RF to skin application patch. FIG. 6A illustrates a rectangular patch 600 that may be used instead of patch 108. The patch is a multilayer structure where one or more conventional electrodes or voltage to skin application elements 604 and 608 are deposited on a substrate 612. (Electrodes 608 and 604 may be identical electrodes, although for convenience of explanation they have been given different numerals.) The voltage to skin application elements or electrodes 604 and 608 may be coated by an electrically conductive biocompatible adhesive. Such adhesive would enable firm electrical and mechanical coupling of the electrode to the skin when the patch is applied to the skin. The other side, of substrate 612 on which electrodes interconnecting pattern is deposited, may be covered by a protective layer, which may be a plastic or paper layer of a desired color or a lacquer layer.

In another embodiment protective layer may be a thermally conductive material, since some of the heat developed at the skin-electrode contact surface will be conducted through the electrodes to the electrodes interconnecting pattern deposited on the opposite to the electrodes side of the substrate. Such material may be aluminum or copper foil, metal powder included in the material of the protective layer. One or more thermal sensors 240 may also be located on the patch. At least one edge of substrate 612 may have an extension 628 or a bay enabling easy and quick connection to RF voltage generator 120 located in case 104 (FIG. 1) by cable 112 or similar cable.

Patch 600 has a layout of electrodes where one or more electrodes 608 located on both sides of electrode 604. The spacing between electrodes 604 and 608 is uniform and electrodes 608 located on both sides of electrode 604 may be located on the same distance from electrode 604. Uniform spacing between the electrodes enables treatment of all skin segments to which the patch is applied at the same skin treatment depth. For treatment of skin layers located at a different skin depth, patches with different spacing or distance between the electrodes may be used or the RF voltage switching order between the electrodes may be changed.

In order to reduce the risk of potential skin overheating, initially RF voltage may be delivered to electrodes 608 and 604 located on one side (for example, on the left or first side of electrode 604 creating in the skin an electric current schematically shown by lines 616-1. As a safety measure temperature sensors 240 may be mounted on patch 600 substrate and used to switch-OFF RF voltage supply.

Upon completion of delivering of RF voltage to the first group of electrodes 604 and 608, RF voltage generator may switch off the first group of electrodes and begin delivery of the RF voltage to another group of electrodes 604 and 608, for example, electrodes located on the right or second side of electrode 604 creating in the skin an electric current schematically shown by lines 616-2. This mode of treatment reduces the risk of skin overheating. The earlier treated segments of skin are thermally relaxing or cooling when the next segment of skin is treated. Coating layer, deposited on the back surface of the electrode assists in reducing temperature of the skin surface.

Referring now to FIGS. 7A-7D which are plan view illustrations of generation of a linear sweeping heating wave effect by conventional RF electrodes 704 similar to electrodes 604 across patch 700 in accordance with an exemplary embodiment of the current apparatus. In FIG. 7A, only the first two electrodes 704 are activated generating a tissue heating effect in a skin segment marked 716-1 that could be located between the electrodes. In FIG. 7B the first two electrodes 704 are inactivated and the next two electrodes 704 are activated generating a tissue heating effect in a skin segment marked 716-2 and so on. Activating electrodes 704 as described in detail above, may create a linear sweeping tissue heating wave effect moving the treated skin segment in a direction indicated by arrows 720.

In FIG. 7C, all previous pairs of electrodes are inactivated and only electrodes 704 of the third pair of electrodes are activated. In FIG. 7D more than one pair of electrodes 704 is activated concurrently. The distance between active electrode pairs may be selected to enable thermal relaxation of the treated tissue.

The treatment protocols including the treatment mode and treatment parameters may be determined earlier in laboratory conditions. Patch electrodes disclosed above are in permanent engagement/contact with skin while the RF current is supplied in pulses pulsed to increase the temperature of the treated skin volume to about 40 - 62 degrees Celsius and maintain it for a certain treatment time, although limiting heating of the skin surface to 40 - 45 degrees Celsius or a lower value. As it is known in the art, methods of cooling both electrodes and skin surface exist and are described elsewhere. Any of these cooling methods may be applied with the present treatment.

FIG. 8 is a simplified illustration of a user performing skin treatment in accordance with the current apparatus. User 800 applies patch 804 which may be any one of the described above patches and places case 104 that includes RF voltage generator 124 (FIG. 1) into a pouch 808 carried, for example, on the user 800 arm 812. The user connects between patch 804 and case 104 by cable 112 and by pushing button 124 (FIG. 1) switches ON RF voltage generator 120. The user could set one of the predetermined skin treatment protocols. RF voltage generator provides test RF voltage and determines the quality of the contact between the patch electrodes and the treated segment of skin. Measurement of the impedance between the electrodes and the skin may serve as the quality of the contact between the patch electrodes and the treated segment of skin indicator. Upon determination of the electrodes status RF voltage generator may begin delivery of the treatment RF voltage to the electrodes of the patch. The treatment continues for the time set by the skin treatment protocol and the user is free address and work on other issues and tasks. If the user desires he can track the treatment process by operation status indicator 128 (FIG. 1) such as an LED that may lit with more than one color.

The employment of an applicator that includes disposable parts for electromagnetic radiation skin treatment simplifies and facilitates aesthetic treatments in a home environment at a time most convenient for the user to perform a treatment session. Use of RF energy for performing skin treatment according to a predetermined treatment protocol is safe and enables a lay user to use it in a residential environment and provides the user with freedom to perform concurrently to the treatment other tasks.

A number of embodiments have been described. Nevertheless, it will be understood that various modifications may be made without departing from the scope of the patch or sheet as defined by the appended claims.

## Claims

1. A patch for personal cosmetic skin treatment, said patch comprising:
a substrate with a plurality of electrodes operative to couple RF voltage to skin when the patch is applied to a treated skin segment; and
wherein the electrodes are coated by an electrically conductive adhesive, said adhesive enabling firm electrical and mechanical coupling of the patch and the electrodes to the treated skin segment;
**characterized in** having an RF voltage supply that is configured to supply RF voltage to a predetermined pairs of electrodes to generate a linear sweeping heating wave effect and wherein heating at least a segment of skin between at least a first pair of electrodes.

2. The patch according to claim 1 wherein the substrate is one of a group of substrates consisting of polyimide film, paper, and plastic material, with a thickness of 0.5mil to 60mil (12.5 micron to 1500 micron).

3. The patch according to claim 1 wherein free of electrodes areas of the substrate are covered by an electrically isolative adhesive.

4. The patch according to claim 1 further comprising light sources configured to deliver light with wavelength of 570 to 780 nm to the treated skin segment.

5. The patch according to claim 1 further comprising one or more temperature sensors operative to switch RF voltage supply if skin or electrode temperature exceeds a required limit.

6. A sheet of disposable patches for personal cosmetic skin treatment, the sheet comprising a plurality of patches according to any one of claims 1 to 5, said sheet further comprising:
a substrate with a plurality of electrodes located on different patch shapes with each patch surrounded by cut-out lines; and
a layer of electrically conductive adhesive covering the electrodes located on the patch shapes.

7. The sheet of disposable patches according to claim 6 further comprising a release layer covering said sheet.

8. The sheet of disposable patches according to claim 6 further comprising cut-outs enabling easy patch separation said cut-outs made in the substrate and in release layer.

9. The sheet of disposable patches according to claim 6 wherein the electrodes are deposited on a substrate which is one of a group of substrates consisting of polyimide film, paper, and plastic material, with a thickness of 0.5mil to 60mil (12.5 micron to 1500 micron).

10. The patch according to claim 1 further comprising a skin treatment protocol selection device operative to set a skin treatment protocol and wherein the skin treatment protocol selection device operation is initiated by configuring the skin treatment protocol selection device.

## Patentansprüche

1. Patch zur persönlichen kosmetischen Hautbehandlung, wobei das Patch Folgendes umfasst:
ein Substrat mit einer Vielzahl von Elektroden, die wirksam sind, um Hochfrequenzspannung an Haut zu koppeln, wenn das Patch auf ein behandeltes Hautsegment aufgebracht ist; und
wobei die Elektroden mit einem elektrisch leitfähigen Haftmittel beschichtet sind, wobei das Haftmittel das feste elektrische und mechanisches Koppeln des Patchs und der Elektroden an das behandelte Hautsegment ermöglicht;
**dadurch gekennzeichnet, dass** es eine Hochfrequenz-Spannungsversorgung aufweist, die dazu konfiguriert ist, Hochfrequenzspannung an ein vorherbestimmtes Paar von Elektroden zu liefern, um einen Effekt einer wärmenden linearen Sägezahnwelle zu erzeugen und wobei mindestens ein Segment von Haut zwischen mindestens einem ersten Paar von Elektroden erwärmt wird.

2. Patch nach Anspruch 1, wobei es sich bei dem Substrat um eines einer Gruppe von Substraten handelt, die aus Polyimidfilm, Papier und Kunststoff mit einer Dicke von 0,5 mil bis 60 mil (12,5 Mikrometer bis 1500 Mikrometer) besteht.

3. Patch nach Anspruch 1, wobei Bereiche des Substrats, die frei von Elektroden sind, mit einem elektrisch isolierenden Haftmittel bedeckt sind.

4. Patch nach Anspruch 1, weiter umfassend Lichtquellen, die dazu konfiguriert sind, Licht mit einer Wellenlänge von 570 bis 780 nm an das behandelte Hautsegment zuzuführen.

5. Patch nach Anspruch 1, weiter umfassend einen oder mehr Temperatursensoren, die wirksam sind, um Hochfrequenz-Spannungsversorgung zu schalten, wenn die Haut- oder Elektrodentemperatur einen erforderlichen Grenzwert übersteigt.

6. Bogen von Einweg-Patches für die persönliche kosmetische Hautbehandlung, wobei der Bogen eine Vielzahl von Patches nach einem der Ansprüche 1 bis 5 umfasst, wobei der Bogen weiter Folgendes umfasst:
ein Substrat mit einer Vielzahl von auf unterschiedlichen Patchformen befindlichen Elektroden, wobei jedes Patch von Ausschneidelinien umgeben ist; und
eine Schicht aus elektrisch leitfähigem Haftmittel, die die auf den Patchformen befindlichen Elektroden bedeckt.

7. Bogen von Einweg-Patches nach Anspruch 6, weiter umfassend eine den Bogen bedeckende Trennschicht.

8. Bogen von Einweg-Patches nach Anspruch 6, weiter umfassend Ausschnitte, die das einfache Trennen von Patches ermöglichen, wobei die Ausschnitte in dem Substrat und in der Trennschicht gebildet sind.

9. Bogen von Einweg-Patches nach Anspruch 6, wobei die Elektroden auf einem Substrat abgeschieden sind, bei dem es sich um eines einer Gruppe von Substraten handelt, die aus Polyimidfilm, Papier und Kunststoff mit einer Dicke von 0,5 mil bis 60 mil (12,5 Mikrometer bis 1500 Mikrometer) besteht.

10. Patch nach Anspruch 1, weiter umfassend eine Hautbehandlungsprotokoll-Auswahlvorrichtung, die wirksam ist, um ein Hautbehandlungsprotokoll einzustellen und wobei der Betrieb der Hautbehandlungsprotokoll-Auswahlvorrichtung durch Konfigurieren der Hautbehandlungsprotokoll-Auswahlvorrichtung ausgelöst wird.

## Revendications

1. Timbre de traitement cutané cosmétique personnel, ledit timbre comprenant :
un substrat comportant une pluralité d'électrodes servant à coupler une tension RF à la peau quand le timbre est appliqué sur un segment de peau traité ; et
dans lequel les électrodes sont revêtues d'un adhésif électriquement conducteur, ledit adhésif permettant un couplage électrique et mécanique ferme du timbre et des électrodes sur le segment de peau traité ;
**caractérisé en ce qu'**il comporte une alimentation de tension RF qui est configurée pour fournir une tension RF à une paire prédéterminée d'électrodes afin de générer un effet de balayage linéaire d'une onde chauffante et dans lequel le chauffage porte sur au moins un segment de peau entre au moins une première paire d'électrodes.

2. Timbre selon la revendication 1, dans lequel le substrat est l'un d'un groupe de substrats consistant en film polyimide, papier, et matière plastique, d'une épaisseur de 0,5 millième de pouce à 60 millièmes de pouce (12,5 microns à 1500 microns).

3. Timbre selon la revendication 1, dans lequel des zones du substrat dépourvues d'électrodes sont recouvertes par un adhésif électriquement isolant.

4. Timbre selon la revendication 1, comprenant en outre des sources de lumière configurées pour délivrer une lumière d'une longueur d'onde de 570 à 780 nm au segment de peau traité.

5. Timbre selon la revendication 1, comprenant en outre un ou plusieurs capteurs de température servant à commuter l'alimentation de tension RF si la température de la peau ou des électrodes dépasse une limite requise.

6. Feuille de timbres jetables de traitement cutané cosmétique personnel, la feuille comprenant une pluralité de timbres selon l'une quelconque des revendications 1 à 5, ladite feuille comprenant en outre :
un substrat comportant une pluralité d'électrodes situées sur différentes formes de timbres, chaque timbre étant entouré par des lignes de découpage ; et
une couche d'adhésif électriquement conducteur recouvrant les électrodes situées sur les formes de timbres.

7. Feuille de timbres jetables selon la revendication 6, comprenant en outre une couche pelable recouvrant ladite feuille.

8. Feuille de timbres jetables selon la revendication 6, comprenant en outre des découpes permettant la séparation aisée des timbres, lesdites découpes étant effectuées dans le substrat et dans la couche pelable.

9. Feuille de timbres jetables selon la revendication 6, dans laquelle les électrodes sont déposées sur un substrat qui est l'un d'un groupe de substrats consistant en film polyimide, papier, et matière plastique d'une épaisseur de 0,5 millième de pouce à 60 millièmes de pouce (12,5 microns à 1500 microns).

10. Timbre selon la revendication 1, comprenant en outre un dispositif de sélection de protocole de traitement cutané servant à établir un protocole de traitement cutané et dans lequel le fonctionnement du dispositif de sélection de protocole de traitement cutané est lancé en configurant le dispositif de sélection de protocole de traitement cutané.
